# EUROPEAN PATENT APPLICATION

(11) **EP 1 939 629 A2**
(43) Date of publication of application: **02.07.2008**
(21) Application number: 07104742.7
(22) Date of filing: 23.03.2007
(51) Int. Cl.: G01N 35/00

(54) **Centrifugal Force Based Magnet Position Control Device and Disk-Shaped Micro Fluidic System**

(30) Priority: 11.08.2006 KR 20060076369; 03.01.2007 KR 20070000703
(71) Applicant: Samsung Electronics Co., Ltd., Suwon-si, Gyeonggi-Do (KR)
(72) Inventor: Lee, Jeong-gun c/o Samsung Adv. Inst. of Tech., Yongin-su Gyeonggi-do (KR); Cho, Yoon-kyoungc/o Samsung Adv. Inst. of Tech., Yongin-su Gyeonggi-do (KR); Lee, Beom-seok c/o Samsung Adv. Inst. of Tech., Yongin-su Gyeonggi-do (KR); Park, Jong-myeon c/o Samsung Adv. Inst. of Tech., Yongin-su Gyeonggi-do (KR)
(74) Representative: Grünecker, Kinkeldey, Stockmair & Schwanhäusser Anwaltssozietät

(57) **Abstract**

A centrifugal force based magnet position control device and a micro fluidic system using the same are provided. The magnet position control device includes: a rotational plate having a guide rail shaped in a path connecting a plurality of positions having different distances from a rotational axis, the guide rail guiding a movement of a first magnet mounted therein; a rotational driver rotating the rotational plate; and a second magnet disposed outside the rotational plate so as to be permanently or temporarily fixed on a position corresponding to a position in the guide rail. The micro fluidic system according to the present invention is configured to control the position of the magnetic bead in the disk-shaped micro fluidic unit disposed to rotate with the rotational plate.

## Description

### BACKGROUND OF THE INVENTION

### 1. Field of the Invention

The present invention relates to a centrifugal force based magnet position control device and a disk-shaped micro fluidic system, and more particularly, to a centrifugal force based magnet position control device that can control the position of a magnet using a centrifugal force at a rotational plate disposed adjacent to a disk-shaped micro fluidic unit provided with a structure for micro flow such that the position of a magnetic bead is desirably controlled in the disk-shaped micro fluidic unit, and a disk-shaped micro fluidic system using the same.

### 2. Description of the Related Art

As a method for separating a targeted bio-molecule from a biological sample, such as blood plasma, methods using silica, glass fiber, anion-exchange resin or magnetic bead are known to those skilled in the art. Among such methods, according to the method using the magnetic bead, a magnetic bead having a probe capable of being combined with a target bio-molecule on a surface thereof is steeped in a sample solution to trap the targeted bio-molecule, and is then separated from the sample solution to extract the targeted bio-molecule. Thus, the bead based separation method has been commercialized and widely used in order to separate cell, protein, nucleic acid, other bio-molecule, or the like. For example, US patent No. 6,893,881 discloses a method for separating specific target cells using a paramagnetic bead coated with an antibody.

Meanwhile, there is recently proposed a micro fluidic system that can perform various reactions in a single unit for the purpose of analysis and processing of sample solution. As one example, a compact disk (CD)-shaped micro fluidic system which moves a sample solution using a centrifugal force in a disk-shaped micro fluidic unit is being developed. Then, to perform a desired work using a magnetic bead in the disk-shaped micro fluidic system, it is required to control the position of the magnetic bead independently from the movement of the sample solution.

### SUMMARY OF THE INVENTION

The present invention provides a centrifugal force based magnet position control device and a disk-shaped micro fluidic system that can control the position of a magnet using a centrifugal force generated by a rotational movement to control the position of a magnetic bead in a microfluidic device included in the microfluidic system independently of movement of a sample solution.

According to an aspect of the present invention, there is provided a centrifugal force based magnet position control device including: a rotational plate having a guide rail shaped in a path connecting a plurality of positions having different distances from a rotational axis, the guide rail guiding a movement of a first magnet mounted therein; a rotational driver rotating the rotational plate; and a second magnet disposed outside the rotational plate so as to be permanently or temporarily fixed on a position corresponding to a position in the guide rail.

The rotational plate may be made of a material which does not have a magnetic property nor block a magnetic force. The first magnet and the second magnet may be disposed such that an attractive force acts therebetween or such that a repulsive force acts therebetween. The second magnet may have a fixed position at least while the rotational driver operates. The above magnet position control device may further include a second magnet moving unit moving the second magnet in a radius direction of the rotational plate.

The first magnet may be a permanent magnet and the second magnet is a permanent magnet or electronic magnet. At this time, the first magnet may be any one selected from the group consisting of a ceramic permanent magnet, a rare-earth permanent magnet, an alnico permanent magnet and a polymer permanent magnet.

The aforementioned magnet position control device may be driven by a method comprising increasing the rotational speed of the rotational plate to increase a centrifugal force acting on the first magnet as a position of the first magnet to be moved in the guide rail is far from the second magnet.

The guide rail may include a path-shaped portion branched into two branches in a distal direction to the rotational axis of the rotational plate from a closest position to the rotational axis of the rotational plate. In this case, the magnet position control device can be driven by a method including: selecting a rotational direction of the rotational plate depending on a direction of one of the two branches where the first magnet is moved; and increasing the rotational speed of the rotational plate to increase a centrifugal force acting on the first magnet as a position of the first magnet to be moved is far from the second magnet.

According to another aspect of the present invention, there is provided a micro fluidic system using a centrifugal force based magnet position control device including: a disk-shaped micro fluidic unit provided with a micro fluidic structure processing a sample solution containing a mixed magnetic bead; a rotational plate disposed adjacent to the micro fluidic unit and having a guide rail shaped in a path connecting a plurality of positions having different distances from a rotational axis, the guide rail guiding a movement of a first magnet mounted therein; a rotational driver rotating the micro fluidic unit and the rotational plate at the same angular velocity; and a second magnet disposed outside the rotational plate so as to be permanently or temporarily fixed on a position corresponding to a position in the guide rail.

The second magnet may be disposed closer to the rotational plate than the micro fluidic unit such that the second magnet has an influence only on the position of the first magnet and does not substantially have an influence on the position of the magnetic bead.

The rotational plate may be made of a material which does not have a magnetic property nor block a magnetic force. The first magnet and the second magnet may be disposed such that an attractive force acts therebetween or such that a repulsive force acts therebetween. The second magnet may have a fixed position at least while the rotational driver operates. Meanwhile, the micro fluidic system of claim may further include a second magnet moving unit moving the second magnet in a radius direction of the rotational plate.

The first magnet may be a permanent magnet and the second magnet is a permanent magnet or electronic magnet. At this time, the first magnet may be any one selected from the group consisting of a ceramic permanent magnet, a rare-earth permanent magnet, an alnico permanent magnet and a polymer permanent magnet.

The aforementioned micro fluidic system may be driven by a method including increasing the rotational speed of the rotational plate to increase a centrifugal force acting on the first magnet as a position where the first magnet is to be disposed so as to control the position of the magnetic bead in the micro fluidic unit is far from the second magnet.

The guide rail may include a path-shaped portion branched into two branches in a distal direction to the rotational axis of the rotational plate from a closest position to the rotational axis of the rotational plate. In this case, the micro fluidic system may be driven by a method including: selecting a rotational direction of the rotational plate depending on a direction of one of the two branches where the first magnet is disposed so as to control the position of the magnetic bead in the micro fluidic unit; and increasing the rotational speed of the rotational plate to increase a centrifugal force acting on the first magnet as a position of the first magnet to be moved is far from the second magnet.

### BRIEF DESCRIPTION OF THE DRAWINGS

The above and other features and advantages of the present invention will become more apparent by describing in detail exemplary embodiments thereof with reference to the attached drawings in which:
FIG. 1 is a plane view of a disk-shaped micro fluidic unit in an embodiment of a disk-shaped micro fluidic system according to the present invention;
FIG. 2 is a plane view of a rotational plate of a magnet position control device in an embodiment of a disk-shaped micro fluidic system according to the present invention;
FIG. 3 is a sectional view taken along line III-III of FIG. 2;
FIG. 4 is a three-dimensional image of the rotational plate of FIG. 2;
FIGS. 5A through 5C show a variety of operation modes of the magnet position control device according to the present invention;
FIG. 6 is a plane view of a base part in an embodiment of a magnet position control device according to the present invention;
FIG. 7 is a plane view of a rotational plate in another embodiment of a magnet position control device according to the present invention;
FIGS. 8 through 10 show various embodiments of a magnet position control device designed integrally with various micro fluidic units according to the present invention;
FIGS. 11A and 11 B are free body diagrams illustrating an external force acting on the first magnet in the embodiment shown in FIGS. 5A through 5C; and
FIG. 12 is a graph showing a relationship between a magnetic force and the horizontal distance shown in FIG. 11 B and a relationship between a centrifugal force and the horizontal distance shown in FIG. 11 B.

### DETAILED DESCRIPTION OF THE INVENTION

The present invention will now be described more fully with reference to the accompanying drawings, in which exemplary embodiments of the invention are shown. The following embodiment illustrates an example of a micro fluidic system including a magnet position control device for controlling a disk-shaped micro fluidic unit and a magnetic bead in the disk-shaped micro fluidic unit. Accordingly, technical features and advantages of a centrifugal based magnet position control device and a micro fluidic system using the same according to the present invention will be understood.

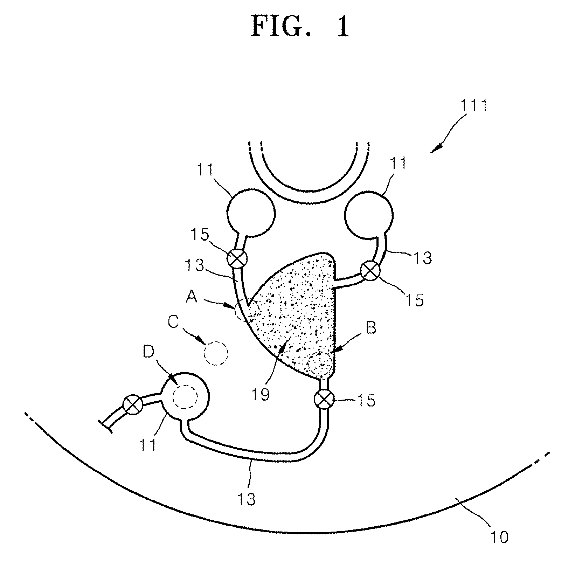

FIG. 1 is a plane view of a disk-shaped micro fluidic unit in an embodiment of a disk-shaped micro fluidic system according to the present invention. The disk-shaped micro fluidic unit 10 shown in FIG. 1 is only a non-limited example of a disk-shaped micro fluidic unit, and any type of disk-shaped micro-fluidic unit known in the related art can be applied to the micro fluidic system of the present invention. Referring to FIG. 1, the micro fluidic unit has a micro fluidic structure 111 disposed on a disk-shaped platform 10. The microfluidic structure 111 includes a plurality of chambers 11, a channel 13 connecting the plurality of chambers 11, and a valve 15 disposed on a mid portion of the channel 13, for controlling a flow of a sample solution. The sample solution in the micro fluidic structure 111 can be transferred by a centrifugal force generated by a rotational movement of the disk-shaped micro fluidic unit 10.

In the micro fluidic system according to the present invention, the micro fluidic structure 111 can mix a magnetic bead 19 with a sample solution to perform a work, such as, a separation of a target bio-molecule. Then, in order to perform a work using the magnetic bead 19, it is necessary to control the position of the magnetic bead 19 in the micro fluidic structure 111, independently of the transfer of the sample solution. For example, when a sample solution is discharged from a chamber in which the sample solution mixed with the magnetic bead is received, while keeping the magnetic bead collected at a corner of the chamber, a buffer solution is filled in the chamber, and the collected magnetic bead is again dispersed to perform a work, such as a cleaning, it is necessary to control the magnetic bead 19 using means other than the centrifugal force. To control the position of the magnetic bead as above, it is necessary to selectively dispose magnets at a variety of positions adjacent to the micro fluidic unit 10 outside the micro fluidic unit 10, for example, at positions A, B, C and D marked in FIG. 1.

Then, since the disk-shaped micro fluidic unit is rotated with respect to a fixed frame, a magnet position control device that can rotate with the platform 10 of the micro fluidic unit and allow magnets to be disposed on the positions A, B, C and D is further needed.

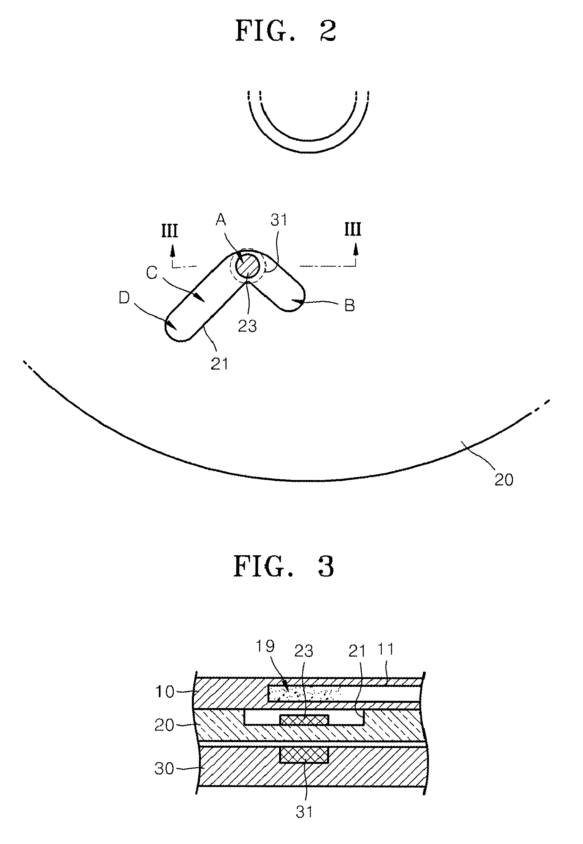

FIG. 2 is a plane view of a rotational plate 20 of a magnet position control device in an embodiment of a disk-shaped micro fluidic system according to the present invention. The rotational plate 20 can be provided with a V-shaped guide rail 21 which is via the positions A, B, C and D. The guide rail 21 is provided in a trench form or channel form on the disk-shaped rotational plate 20 and serves as a moving passage along which a first magnet 23 installed therein moves. The guide rail 21 can have various shapes, for example, U-shape as well as V-shape, depending on positions where the first magnet 23 needs to stay. That is, the guide rail 21 may have any shape if it functions to connect a variety of positions where the first magnet 23 needs to stay, having different distances from a rotational axis of the rotational plate 20.

Meanwhile, the second magnet 31 may be disposed outside the rotational plate 20 so as to be temporarily fixed on a position corresponding to a position that is the closest to the rotational axis in a moving path of the first magnet or so as to be permanently fixed on a position corresponding to a position that is the closest from the rotational axis in the guide rail 21. In the case of the V-shaped guide rail 21 as shown in FIG. 2, it can be branched into two passages toward a distal direction from the rotational axis, starting from the closest position A to the rotational axis. In this case, it is preferable that the second magnet 31 be fixedly disposed at a position corresponding to the closest position A to the rotational axis at least while the rotational plate 20 rotates.

FIG. 3 is a sectional view taken along line III-III of FIG. 2, and shows a vertical structure of the micro fluidic system including a platform 10 of the micro fluidic unit, the rotational plate 20 having the first magnet 23 mounted thereon, and the second magnet 31 disposed on a base part 30. The rotational plate 20 is disposed below the platform 10, and the second magnet 31 is disposed below the rotational plate 20. The second magnet 31 and the first magnet 23 are preferably arranged such that poles opposite to each other face for an interaction of an attractive force therebetween. Alternatively, the second magnet 31 and the first magnet 23 may be arranged such that poles having the same polarity face for an interaction of a repulsive force therebetween. In the case of the latter, it is noted that the position of the second magnet 31 should be made different from the position shown in FIG. 2. Also, the second magnet 31 has a magnetic flux density to such a degree that it does not have a direct influence on the magnetic bead 19 in the chamber 11 and only the first magnet 23 has a direct influence on the magnetic bead 19. Herein, the direct influence means the influence to such a degree that the magnetic beads 19 can be focused or moved by the magnetic force.

The first magnet 23 is preferably a permanent magnet. More specifically, the first magnet 23 can be a permanent magnet selected from the group consisting of a ceramics permanent magnet group including barium ferrite, strontium ferrite and the like, a rare-earth permanent magnet group including samarium-cobalt, neodium and the like, an alloy permanent magnet group including alnico (aluminum-nickel-cobalt) and the like, and a polymer-based magnet group including rubber magnet and plastic magnet. For miniaturization of the micro fluidic system, it is preferable that the first magnet 23 has a small size and a large magnetic force. In the present embodiment, a neodium magnet is used as the first magnet. The second magnet 31 may be a permanent magnet or an electronic magnet. Since the second magnet 31 is equipped in a fixed frame, it has a less limitation in size.

Any type of the magnetic bead 19 can be used in the micro fluidic system according to the present invention if it is a fine particle having a magnetic property. In general, it is preferable that the magnetic bead 19 has a diameter ranging from 0.001 µm to 100 µm, more preferably, 0.1 µm to 100 µm. The size of the magnetic bead 19 can be properly selected depending on the size of a target bio-molecule to be separated and filtered through the micro fluidic system. Also, the magnetic bead 19 can be formed of any material if it has a magnetic property. In particular, the magnetic bead 19 can be made of a metal, such as iron (Fe), nickel (Ni), chromium (Cr), cobalt (Co), and a metal oxide thereof showing the ferromagnetic property. Additionally, the magnetic bead 19 may include a material, for example, gadolinium (Gd), terbium (Tb), diprosium (Dy), erbium (Er), holmium (Ho) or the like.

The substrate for the rotational plate 20 and the micro fluidic unit 10 is preferably made of a material which does not have a magnetic property and not shield the magnetic force. This is because the first magnet 23, the second magnet 31 and the magnet bead 19 as made of such a material show a smooth movement through the magnetic force.

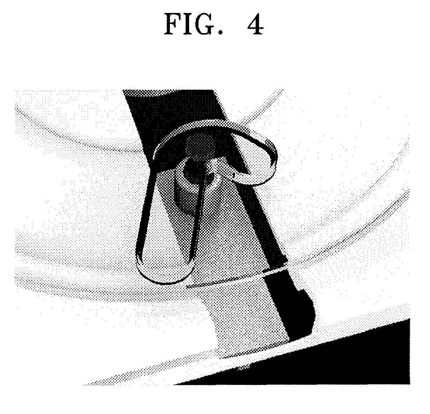

FIG. 4 is a three-dimensional image of the rotational plate of FIG. 2, and is a rendering image to help a visual understanding of a relationship between the first magnet 23 and the second magnet 31.

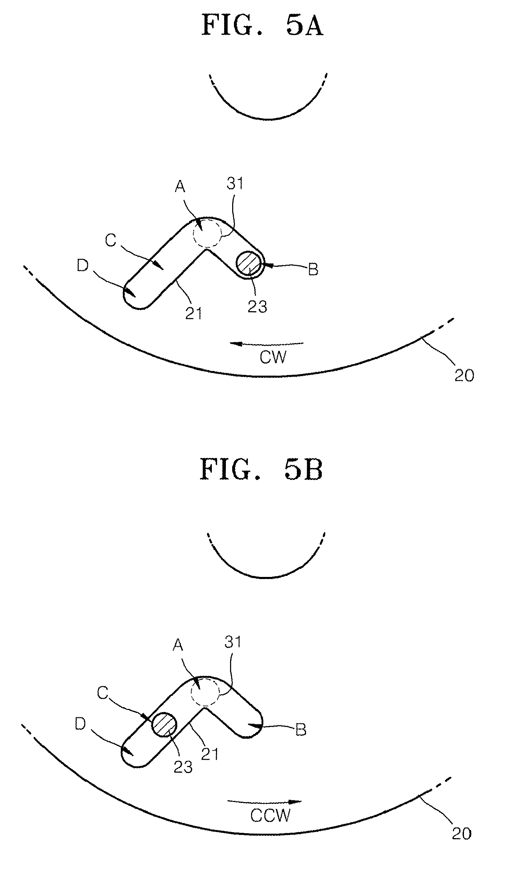

FIGS. 5A through 5C show a variety of operation modes of the magnet position control device according to the present invention. First, FIG. 5A shows a mode that the rotational plate 20 rotates at a rotational speed of 100 rpm clockwise (CW). As aforementioned, the second magnet 31 having the fixed position is disposed corresponding to the position A closest to the rotational axis. When the rotational plate 20 is accelerated clockwise, the first magnet 23 is biased toward the right of the drawing due to an inertial force. As the rotational speed of the rotational plate 20 increases, the first magnet 23 moves to the position B where an attractive force from the second magnet 31 is equilibrated with a centrifugal force. While the rotational speed of the rotational plate 20 is kept, the first magnet 23 stays at the position B, and as the rotational speed decreases, the first magnet 23 returns to the position A. At this time, if the disk-shaped micro fluidic unit (see FIG. 1) rotates on the rotational plate 20 together with the rotational plate 20, the magnetic bead 19 is collected at the position B shown in FIG. 1. In the above, the rotational speed of 100 rpm is only one example, and it can be varied with the magnetic flux density of the first and second magnets 23 and 31 and a distance between the positions A and B. The above fact is equally applied to the following modes.

FIG. 5B shows a mode that the rotational plate 20 rotates at a rotational speed of 150 rpm counterclockwise (CCW). When the rotational plate 20 is accelerated counterclockwise, the first magnet 23 is biased toward the left of the drawing due to an inertial force. As the rotational speed of the rotational plate 20 increases, the first magnet 23 moves to the position C where an attractive force from the second magnet 31 is equilibrated with a centrifugal force. While the rotational speed of the rotational plate 20 is kept, the first magnet 23 stays at the position C, and as the rotational speed decreases, the first magnet 23 returns to the position A. At this time, if the disk-shaped micro fluidic unit (see FIG. 1) rotates on the rotational plate 20 together with the rotational plate 20, the magnetic bead 19 in the chamber 11 keeps a dispersed state without being influenced by the first magnet 23.

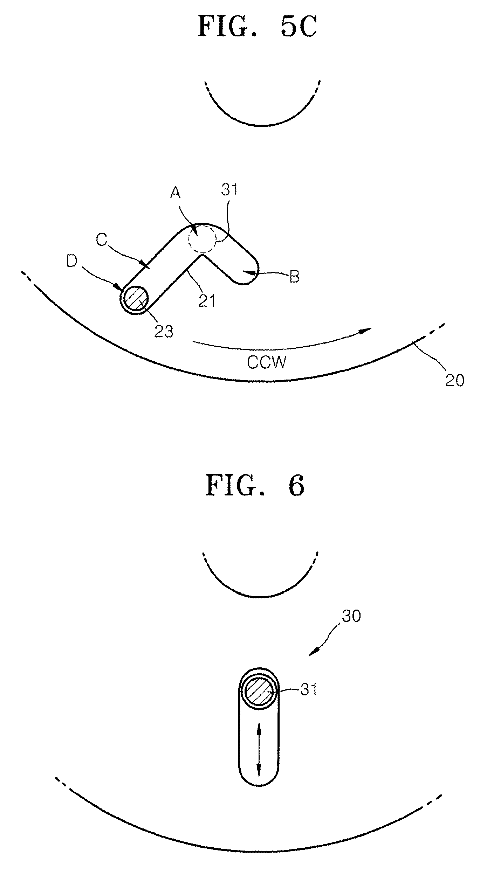

FIG. 5C shows a mode that the rotational plate 20 rotates at a rotational speed of 1000 rpm counterclockwise (CCW). Likewise, when the rotational plate 20 is accelerated counterclockwise, the first magnet 23 is biased toward the left of the drawing due to an inertial force. As the rotational speed of the rotational plate 20 increases, the first magnet 23 moves to a distal position from the second magnet 31. As the rotational speed of the rotational plate 20 increases, the centrifugal force increases so that the first magnet 23 moves to a distal position from the rotational axis, and as the rotational speed decreases, the first magnet 23 returns to the original position A. Then, since an attractive force between the first magnet 23 and the second magnet 31 decreases as a distance therebetween increases, at the rotational speed above a predetermined level, the first magnet 23 moves to and stays at the position D where the guide rail 21 is ended. At this time, if the disk-shaped micro fluidic unit (see FIG. 1) rotates on the rotational plate 20 together with the rotational plate 20, the magnetic bead 19 is collected in the chamber 11 at the position D.

FIG. 6 is a plane view of a base part in an embodiment of a magnet position control device according to the present invention. In FIG. 6, the base part 30 is a part of the fixed frame in the micro fluidic system of the present invention, and the rotational plate 20 and the micro fluidic unit are disposed on the base part 30. The aforementioned construction corresponds to an example that the rotational plate is disposed below the micro fluidic unit, but it is different from an example that the rotational plate is disposed on the micro fluidic unit.

The second magnet 31 which can be temporarily fixed on the base part 30 is fixed at least while the rotational plate rotates. However, like in the mode shown in FIG. 5C, after the first magnet 23 gets out of an attractive force range of the second magnet 31 fixed at one position, a second magnet moving unit (not shown) allows the first magnet 23 to be moved in a radius direction so as to attract the first magnet within its attractive force range. The second magnet moving unit is allowable if it can be installed on the base part 30 which is a fixed frame, to move the second magnet 31 along a straight line. Accordingly, while a detailed description for the second magnet moving unit is not given in detail in the present specification, the second magnet moving unit can be easily implemented by those skilled in the art.

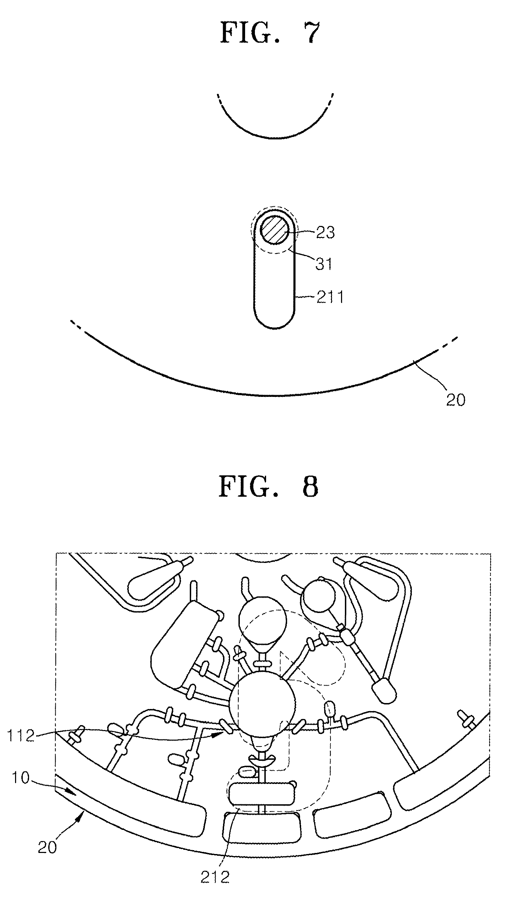

FIG. 7 is a plane view of a rotational plate in another embodiment of a magnet position control device according to the present invention. As aforementioned, a guide rail 211 is a path connecting the positions where a first magnet 23 is temporarily disposed. The guide rail 211 may have various shapes or a simple straight line shape like the guide rail 211 shown in FIG. 7.

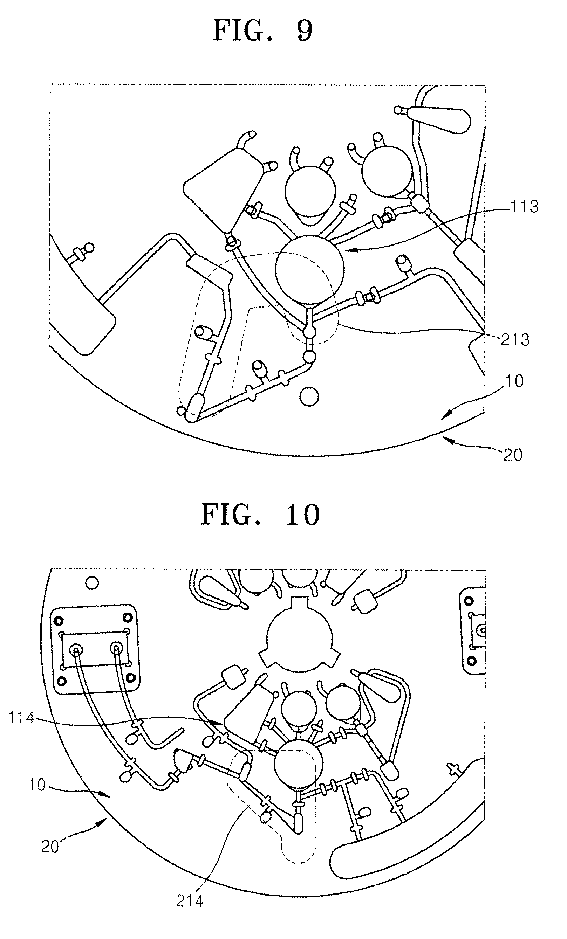

FIGS. 8 through 10 show various embodiments of a magnet position control device designed integrally with various micro fluidic units according to the present invention. According to the embodiments of FIGS. 8 through 10, each of micro fluidic structures 112, 113, 114 suitably configured to have an inherent function is disposed on a platform 10 of a micro fluidic unit. A rotational plate 20 of the magnet position control device according to the present invention is integrally provided below the platform 10. Guide rail 212, 213, 214 having different shapes with one another are respectively disposed in the corresponding rotational plates 20. The respective guide rails 212, 213, 214 provide a path to collect or move a magnetic bead in the respective micro fluidic structures 112, 113, 114 independent of movement of fluid by a centrifugal force, the path connecting a plurality of positions having different distances from a rotational axis. Although not shown in FIGS. 8 through 10, a first magnet (not shown) is mounted on each of the guide rails 212, 213, 214. The position of the first magnet (not shown) is controlled by i) a centrifugal force by a rotation of the platform 10 and the rotational plate 20, and ii) a magnetic force between the first magnet and the second magnet (not shown) disposed in a base part (not shown).

In the aforementioned embodiments, the platform 10 of the micro fluidic unit and the rotational plate 20 of the magnet position control device can be attached in an up and down direction as shown in FIG. 3. However, the platform 10 being integrally provided with the rotational plate 20 is not limited to the platform 10 and the rotational plate 20 being independently prepared and attached. For example, the platform 10 and the rotational plate 20 may be one member, the guide rails 212, 213, 214 may be formed in a trench shape on opposite faces to faces where the micro fluidic structures 112, 113, 114 are formed, and a bottom cover (not shown) may be further provided such that the first magnet (not shown) may not fall down from the guide rails 212, 213, 214.

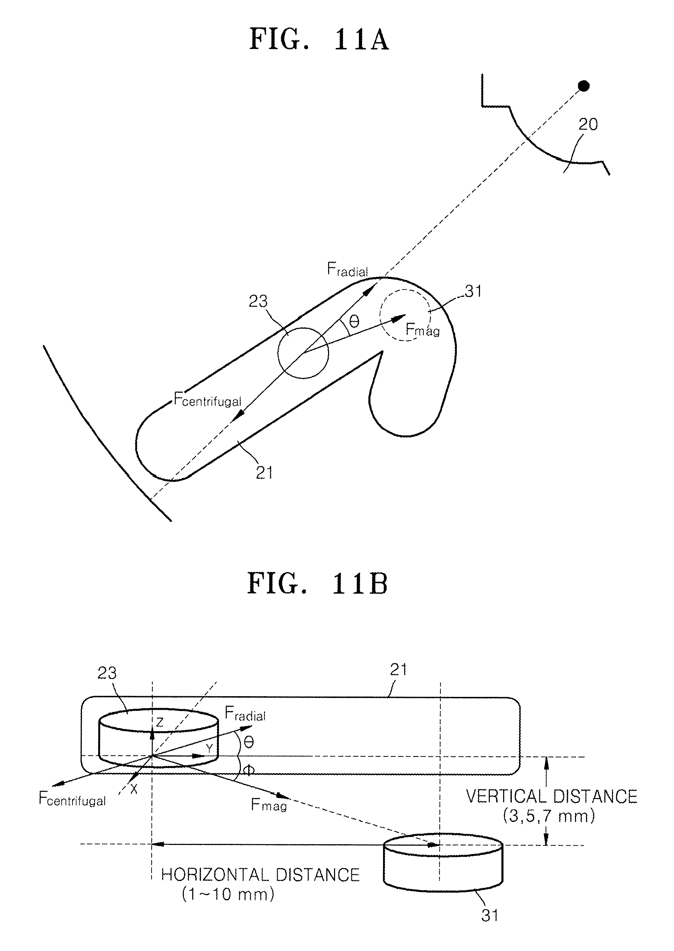

FIGS. 11A and 11 B are free body diagrams illustrating an external force acting on the first magnet in the embodiment shown in FIGS. 5A through 5C. When the guide rail 21 has the same shape as that shown in FIGS. 11A and 11B, a force acting on the first magnet 23 can be obtained as follows.

First, a centrifugal force acting on the first magnet 23, F_{cent} = mrω²,
where m is a weight of the first magnet 23, r is a distance from a rotational center, and ω is an angular velocity. The centrifugal force, F_{cent} is parallel with a radial component F_{radial} of a magnetic force by the second magnet 31.

Here, |**F**_{radial}| = |**F**_{mag}| × cos(θ) × cos(φ).

Meanwhile, **F**_{mag}= ∇ U_{mag}, and an instant energy density (U_{mag}) of a magnetic force is given by an equation U_{mag}=1/2**B** · **H.** Nd-Fe-B magnets employed as the first and second magnets 23 and 31 in the present embodiment have physical properties: µ r (relative permiability) is 1.04457301671; σ (conductivity) is 6.25*10⁵[siemens/meter]; Hc (magnetic coercivity) is -8.38*10⁵[Ampere/meter]; Br (magnetic retentivity) is 1.1[Tesla]; and M (magnetization) is 875352.188006[Ampere/meter].

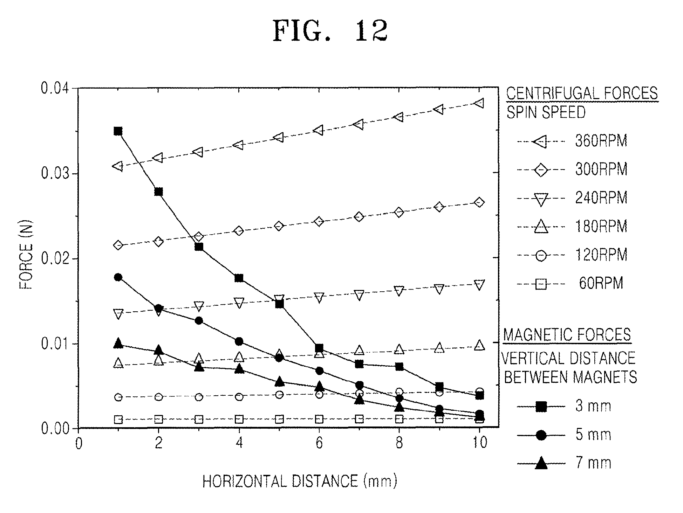

FIG. 12 is a graph showing a relationship between a magnetic force and the horizontal distance shown in FIG. 11 B and a relationship between a centrifugal force and the horizontal distance shown in FIG. 11 B. When the above physical properties of the first and second magnets 23 and 31 are applied, a horizontal distance between the first and second magnets 23 and 31 in an equilibrium state according to a rotational velocity of the rotational plate 20 and a vertical distance between the first and second magnets 23 and 31 has results shown in FIG. 12.

For example, when the rotational velocity is 300 rpm and the vertical distance is 3 mm, a magnetic force wins a centrifugal force until the horizontal distance reaches 3 mm. When the horizontal distance exceeds 3 mm, the centrifugal force is larger than the magnetic force. Although not shown in the graph, when the rotational velocity exceeds 420 rpm, the centrifugal force is always larger than the magnetic force. At this time, the first magnet 23 gets out of a range influenced by the second magnet 31 and moves to the most distal position in the guide rail 21 from the rotational axis.

As described above, according to the present invention, the position of the magnet is controlled two-dimensionally using a centrifugal force due to a rotational movement in a disk-shaped micro fluidic system, thus controlling the position of the magnetic bead independently of a movement of a sample solution by the centrifugal force.

Also, since the position of the magnet is controlled using a rotational movement which is the same as that of a disk-shaped micro fluidic unit having a micro fluidic structure, the present invention is advantageous in the miniaturization and simplification of the disk-shaped micro fluidic system including the magnet position control device.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A centrifugal force based magnet position control device comprising:
a rotational plate having a guide rail shaped in a path connecting a plurality of positions having different distances from a rotational axis, the guide rail guiding a movement of a first magnet mounted therein;
a rotational driver rotating the rotational plate; and
a second magnet disposed outside the rotational plate so as to be permanently or temporarily fixed on a position corresponding to a position in the guide rail.

2. The centrifugal force based magnet position control device of claim 1, wherein the rotational plate is made of a material which does not have a magnetic property nor block a magnetic force.

3. The centrifugal force based magnet position control device of claim 1, wherein the rotational plate has a compact disk shape.

4. The centrifugal force based magnet position control device of claim 1, wherein the first magnet and the second magnet are disposed such that an attractive force acts therebetween.

5. The centrifugal force based magnet position control device of claim 1, wherein the first magnet and the second magnet are disposed such that a repulsive force acts therebetween.

6. The centrifugal force based magnet position control device of claim 1, wherein the second magnet has a fixed position at least while the rotational driver operates.

7. The centrifugal force based magnet position control device of claim 1, further comprising a second magnet moving unit moving the second magnet in a radius direction of the rotational plate.

8. The centrifugal force based magnet position control device of claim 1, wherein the guide rail comprises a path-shaped portion branched into two branches in a distal direction to the rotational axis of the rotational plate from a closest position to the rotational axis of the rotational plate.

9. The centrifugal force based magnet position control device of claim 1, wherein the first magnet is a permanent magnet and the second magnet is a permanent magnet or electronic magnet.

10. The centrifugal force based magnet position control device of claim 9, wherein the first magnet is any one selected from the group consisting of a ceramic permanent magnet, a rare-earth permanent magnet, an alnico permanent magnet and a polymer permanent magnet.

11. A method of driving the magnet position control device of claim 1, the method comprising increasing the rotational speed of the rotational plate to increase a centrifugal force acting on the first magnet as a position of the first magnet to be moved in the guide rail is far from the second magnet.

12. A method of driving the magnet position control device of claim 8, the method comprising:
selecting a rotational direction of the rotational plate depending on a direction of one of the two branches where the first magnet is moved; and
increasing the rotational speed of the rotational plate to increase a centrifugal force acting on the first magnet as a position of the first magnet to be moved is far from the second magnet.

13. A micro fluidic system using a centrifugal force based magnet position control device comprising:
a micro fluidic unit provided on a disk-shaped platform with a micro fluidic structure processing a sample solution containing a mixed magnetic bead;
a rotational plate disposed adjacent to the micro fluidic unit and having a guide rail shaped in a path connecting a plurality of positions having different distances from a rotational axis, the guide rail guiding a movement of a first magnet mounted therein;
a rotational driver rotating the micro fluidic unit and the rotational plate at the same angular velocity; and
a second magnet disposed outside the rotational plate so as to be permanently or temporarily fixed on a position corresponding to a position in the guide rail.

14. The micro fluidic system of claim 13, wherein the second magnet is disposed closer to the rotational plate than the platform of the micro fluidic unit.

15. The micro fluidic system of claim 13, wherein the rotational plate is made of a material which does not have a magnetic property nor block a magnetic force.

16. The micro fluidic system of claim 13, wherein the first magnet and the second magnet are disposed such that an attractive force acts therebetween.

17. The micro fluidic system of claim 13, wherein the second magnet has a fixed position at least while the rotational driver operates.

18. The micro fluidic system of claim 13, further comprising a second magnet moving unit moving the second magnet in a radius direction of the rotational plate.

19. The micro fluidic system of claim 13, wherein the guide rail comprises a path-shaped portion branched into two branches in a distal direction to the rotational axis of the rotational plate from a closest position to the rotational axis of the rotational plate.

20. The micro fluidic system of claim 13, wherein the first magnet is a permanent magnet and the second magnet is a permanent magnet or electronic magnet.

21. The micro fluidic system of claim 20, wherein the first magnet is any one selected from the group consisting of a ceramic permanent magnet, a rare-earth permanent magnet, an alnico permanent magnet and a polymer permanent magnet.

22. A method of driving the micro fluidic system of claim 13, the method comprising increasing the rotational speed of the rotational plate to increase a centrifugal force acting on the first magnet as a position where the first magnet is to be disposed so as to control the position of the magnetic bead in the micro fluidic unit is far from the second magnet.

23. A method of driving the micro fluidic system of claim 19, the method comprising:
selecting a rotational direction of the rotational plate depending on a direction of one of the two branches where the first magnet is disposed so as to control the position of the magnetic bead in the micro fluidic unit; and
increasing the rotational speed of the rotational plate to increase a centrifugal force acting on the first magnet as a position of the first magnet to be moved is far from the second magnet.
